# EUROPÄISCHE PATENTSCHRIFT

(11) **EP 3 258 794 B1**
(45) Veröffentlichungstag und Bekanntmachung des Hinweises auf die Patenterteilung: **06.04.2022**
(21) Anmeldenummer: 15714760.4
(22) Anmeldetag: 20.02.2015
(51) Int. Cl.: A23L 2/02, A23L 2/38, A23L 2/84, C12N 1/20, C12N 9/04, C12M 1/04, C12M 1/34

(54) **VERFAHREN UND VORRICHTUNG ZUR BIOTECHNOLOGISCHEN REDUZIERUNG VON ZUCKERSTOFFEN IN FRUCHTEDUKTEN ZWECKS ERHALTS ZUCKERREDUZIERTER FRUCHTPRODUKTE**
METHOD AND DEVICE FOR THE BIOTECHOLOGICAL REDUCTION OF SUGARS IN FRUIT EDUCTS FOR THE PURPOSE OF OBTAINING REDUCED-SUGAR FRUIT PRODUCTS
PROCÉDÉ ET DISPOSITIF POUR LA RÉDUCTION BIOTECHNOLOGIQUE DES SUCRES DANS LES MATIÈRES PREMIÈRES DE FRUITS DANS LE BUT D'OBTENIR DES PRODUITS DE FRUITS À TENEUR EN SUCRES RÉDUITE

(43) Veröffentlichungstag der Anmeldung: 27.12.2017
(73) Patentinhaber: Loibl Verwaltungs- GmbH, 85354 Freising (DE)
(72) Erfinder: LÖTZBEYER, Thomas, 85402 Kranzberg (DE); JÄGER, Astrid, 85354 Freising (DE); WESTERMEIER, Rosina, 85410 Haag an der Amper (DE)
(74) Vertreter: Strych, Sebastian
(86) Internationale Anmeldenummer: PCT/DE2015/100071
(87) Internationale Veröffentlichungsnummer: WO 2016/131432

(56) Entgegenhaltungen:
- WO-A1-93/14650
- WO-A1-2012/059554
- CH-A5- 668 887
- DE-A1- 2 214 442
- Gary James Pickering: "THE PRODUCTION OF REDUCED-ALCOHOL WINE USING GLUCOSE OXIDASE", Ph. D. thesis, 1997, Seiten i-xvii,1-214, XP055225403, Gefunden im Internet: URL:https://researcharchive.lincoln.ac.nz/ bitstream/handle/10182/1979/pickering_phd. pdf?sequence=5 [gefunden am 2015-11-03]
- AZIZ M G ET AL: "Biotransformation of pineapple juice sugars into dietetic derivatives by using a cell free oxidoreductase from Zymomonas mobilis together with commercial invertase", ENZYME AND MICROBIAL TECHNOLOGY, STONEHAM, MA, US, Bd. 48, Nr. 1, 24. September 2010 (2010-09-24), Seiten 85-91, XP027476954, ISSN: 0141-0229, DOI: 10.1016/J.ENZMICTEC.2010.09.012 [gefunden am 2010-09-24]
- N. V. NARENDRANATH ET AL: "Relationship between pH and Medium Dissolved Solids in Terms of Growth and Metabolism of Lactobacilli and Saccharomyces cerevisiae during Ethanol Production", APPLIED AND ENVIRONMENTAL MICROBIOLOGY, Bd. 71, Nr. 5, Mai 2005 (2005-05), Seiten 2239-2243, XP055223336, US ISSN: 0099-2240, DOI: 10.1128/AEM.71.5.2239-2243.2005
- LEIGH M. SCHMIDTKE ET AL: "Production Technologies for Reduced Alcoholic Wines", JOURNAL OF FOOD SCIENCE, Bd. 77, Nr. 1, 10. November 2011 (2011-11-10), Seiten R25-R41, XP055225476, US ISSN: 0022-1147, DOI: 10.1111/j.1750-3841.2011.02448.x
- G.J PICKERING ET AL: "Optimising glucose conversion in the production of reduced alcohol wine using glucose oxidase", FOOD RESEARCH INTERNATIONAL., Bd. 31, Nr. 10, Dezember 1998 (1998-12), Seiten 685-692, XP055225212, GB ISSN: 0963-9969, DOI: 10.1016/S0963-9969(99)00046-0
- Leif Poll: "Evaluation of 18 apple varieties for their suitability for juice production", JOURNAL OF THE SCIENCE OF FOOD AND AGRICULTURE, vol. 32, no. 11, 1 November 1981 (1981-11-01), pages 1081-1090, XP55653962, GB ISSN: 0022-5142, DOI: 10.1002/jsfa.2740321107
- Leif Poll: "Evaluation of 18 apple varieties for their suitability for juice production", JOURNAL OF THE SCIENCE OF FOOD AND AGRICULTURE, vol. 32, no. 11, 1 November 1981 (1981-11-01), pages 1081-1090, XP055653962, GB ISSN: 0022-5142, DOI: 10.1002/jsfa.2740321107

## Beschreibung

### Gebiet der Erfindung

Die vorliegende Erfindung betrifft ein Verfahren zur biotechnologischen Reduzierung von Zuckerstoffen in Fruchtedukten zwecks Erhalts
zuckerreduzierter Ganzfruchtgetränkte oder Obst- und/oder Gemüsesäfte

### Definitionen

Vorbeugend zu etwaigen Abgrenzungsfragen seien zumindest die nachfolgenden Begriffe eingangs definiert:
So werden mit "*Frucht*" (englisch: "progeny") im Kontext der vorliegenden Erfindung essbare Pflanzenteile bezeichnet, die gemäß unterschiedlicher Definitionen einerseits dem Obst (englisch: "fruit") und andererseits dem Gemüse (englisch: "vegetable") zugeordnet sind:
- Lebensmitteldefinition: Obst stammt von mehrjährigen Pflanzen, Gemüse stammt von einjährigen Pflanzen.
- Botanische Definition: Obst sind aus bestäubten Blüten entstandene Früchte, Gemüse sind andere Pflanzenteile (z. B. Blatt, Wurzel, Knolle usw.).

In der Regel stammt das Obst von mehrjährigen und das Gemüse von einjährigen Pflanzen (Lebensmitteldefinition) ab. Der Zuckergehalt beim Obst ist meist höher. Botanisch gesehen entsteht Obst aus der befruchteten Blüte (z.B.: Ananas, Apfel, Aprikose, Banane, Birne, Erdbeere, Grapefruit, Himbeere, Johannisbeere, Kirsche, Kiwi, Mirabelle, Orange, Pfirsich, Pflaume, Sauerkirsche, Traube, Zitrone, Zwetschge, ...). Gemüse entsteht aus anderen Pflanzenteilen. Gurken, Kürbisse, Paprika, Tomaten und Zucchini sind zwar Früchte und gehören laut der obigen (botanischen) Definition zu Obst (befruchtete Blüte), werden aber als einjährige Pflanzen (Lebensmitteldefinition: Gemüse) und gemeinhin wegen der fehlenden Süße bzw. Säure als Gemüse bezeichnet. Rhabarber hingegen ist ein Blattstiel, wird aber auch als Obst verwendet. Schon insoweit wird verständlich, dass in Literatur und Praxis die Unterscheidung zwischen Obst und Gemüse mitunter unscharf ausfällt.

Unter "***Fruchtedukte***" werden im Kontext der vorliegenden Erfindung solche Ausgangsstoffe verstanden, die aus zerkleinerten Früchten bestehenden. Diese können insbesondere püriert, passiert oder verflüssigt sein. Teile der Früchte wie insbesondere Zellkomponenten können dabei auch entfernt sein.

Des Weiteren werden unter "***zuckerreduzierte Fruchtprodukte"*** im Kontext der vorliegenden Erfindung

Ganzfruchtgetränke (Smoothies) oder Obst- und/oder Gemüsesäfte (gleich ob unverdünnt als sog. Direktsaft oder verdünnt als sog. Fruchtsaft aus Fruchtsaftkonzentrat abgefüllt) verstanden.

Schließlich wird unter "***Zuckerstoffen***" im Kontext der vorliegenden Erfindung die Gruppe aller Kohlenhydrate verstanden, die zu den Mono- und Disacchariden gehören, aber auch längerkettige Kohlenhydrate, die einen süßen Geschmack aufweisen. Beispielhaft zu nennen sind hier Glucose, Galactose, Mannose, Fructose, Saccharose, Lactose oder Maltose.

### Hintergrund der Erfindung

Gerade im Hinblick auf die zahnschädigende Wirkung und eine signifikante Zunahme des Übergewichts und damit einhergehend auch der Fälle von Typ II Diabetes bereits im Kindesalter gibt es vielfache Bestrebungen, die Aufnahmemengen von Zuckerstoffen bei Kindern zu verringern.

Vor diesem Hintergrund ist die Lebensmittelindustrie ständig auf der Suche nach Innovationen. Insbesondere Fruchtprodukte wie Ganzfruchtgetränkte (Smoothies) oder Obst- und/oder Gemüsesäfte (gleich ob unverdünnt als sog. Direktsaft oder rückverdünnt als sog. Fruchtsaft aus Fruchtsaftkonzentrat abgefüllt) oder vergleichbare als alkoholfrei kennzeichenbare Fruchtgetränke enthalten viele gesundheitlich relevante Inhaltsstoffe, können allerdings beim regelmäßigen Verzehr größerer Mengen aufgrund ihres üblicherweise meist hohen Gehalts an Zuckerstoffen wiederum insbesondere bei Kindern zu unerwünschten Gesundheitsbeeinträchtigungen führen.

So macht der natürliche Anteil an Zuckerstoffen in der Trockenmasse beispielsweise in einer Ananas 82 %, in einem Apfel 68 %, in einer Erdbeere 55 %, in einer Grapefruit 67 %, in einer Himbeere 27 %, in einer Johannisbeere 37 %, in einer Orange 66 %, in einer Sauerkirsche 69 % oder in einer Traube 79 % aus.

Die Zuckerstoffe setzen sich dabei in erster Linie aus der Saccharose, der Glucose und der Fructose zusammen, wobei sich deren prozentualen Gesamtmasse-Anteile (also die Masse der Inhaltsstoffe pro Gesamtmasse in %; Einheit: [w/w %]), wie die nachstehende Tabelle zeigt, je nach Frucht stark unterscheiden können.

| **Frucht** | **Wassergehalt [w/w %]** | **Saccharose [w/w %]** | **Glucose [w/w %]** | **Fructose [w/w %]** |
|---|---|---|---|---|
| | | | | |
| Ananas | 85 | 7,8 | 2,1 | 2,4 |
| Apfel | 85 | 2,6 | 1,7 | 5,9 |
| Erdbeere | 90 | 1,0 | 2,2 | 2,3 |
| Grapefruit | 89 | 2,8 | 2,3 | 2,3 |
| Himbeere | 85 | 0,2 | 1,8 | 2,0 |
| Johannisbeere | 81 | 0,7 | 2,7 | 3,6 |
| Orange | 86 | 3,8 | 2,5 | 2,9 |
| Sauerkirsche | 84 | 0,5 | 4,8 | 5,8 |
| Traube | 81 | 0,4 | 7,3 | 7,3 |
| | | | | |

Der Brennwert von Früchten wird somit fast ausschließlich von den darin enthaltenen Zuckerstoffen bestimmt. Daher wäre es wünschenswert, mit einer Entfernung oder Umwandlung der Zuckerstoffe in den aus Früchten erhaltenen Fruchtedukten den Brennwert und zusätzlich die kariogene Wirkung von Fruchtprodukten maßgeblich zu senken.

Diesbezüglich werden im Stand der Technik vornehmlich aufwändige Verfahren zur Isolation von Zuckerstoffen mittels Extraktion oder der Vergärung von Zucker zu Alkohol und CO₂ vorgeschlagen.

So offenbart die DE 2 214 442 A (= GB 1 373 562 B) ein enzymatisches Verfahren zur Überführung von Glucose in Gluconsäure durch Oxidation mit Sauerstoff in wässriger Lösung; wobei eine glucosehaltige wässrige Lösung über einen Katalysator geleitet wird, der Glucoseoxydase und Katalase in unmittelbarer Nachbarschaft gemeinsam auf einem geeigneten Träger gebunden enthält; und wobei der pH-Wert während der Umsetzung zwischen 3,5 und 8,0 gehalten wird.

Des Weiteren offenbart die EP 0 554 488 A1 ein fermentatives Verfahren zur selektiven Verminderung des Zuckergehalts von zuckerhaltigen Nahrungsmitteln unter Beibehaltung der originären sensorischen Eigenschaften und danach erhaltene Nahrungsmittel, wobei das originäre Nahrungsmittel einer kontrollierten Fermentation mit einer Mikroorganismenspezies unterworfen, und wobei aus verschiedenen für Zuckerabbau geeigneten Mikroorganismenspezies die für das jeweilige Nahrungsmittel am besten geeignete Spezies anhand sensorischer Analyse gemäß ISO-Richtlinie 8587 ausgewählt wird. Die damit erhaltenen Nahrungsmittel weisen gegenüber dem originären Ausgangsprodukt einen verminderten Zuckergehalt auf mit Kennzahlen für die restlichen Inhaltsstoffe, die im Bereich der natürlichen Schwankungsbreiten der charakteristischen Zusammensetzung des jeweiligen originären Nahrungsmittels liegen.

Darüber hinaus offenbart die US 4 675 191 A (= AT 43 630 B) ein Verfahren zur Herstellung eines alkoholarmen Weines, wobei zunächst unfermentierter Traubensaft unter Zufuhr von Sauerstoff zu dem Traubensaft mit einer Glucoseoxidase-Präparation behandelt wird, bis wenigstens eine geringere Menge der Glucose in Gluconsäure umgewandelt wird, woraufhin der so behandelte, glucosearme Traubensaft unter Erzeugung des alkoholarmen Weins fermentiert wird, wobei - wenn erwünscht - die Gluconsäure wenigstens teilweise entfernt wird, bis geeignete organoleptische Eigenschaften erzielt sind.

Des Weiteren offenbart die WO 2005/074716 A1 (= EP 1 713 351 B1) ein Verfahren zur Herstellung eines Lebensmittelprodukts durch Hitzebehandlung eines Lebensmittelmaterials, das reduzierende Zucker enthält, umfassend den Schritt, das Lebensmittelmaterial zu blanchieren, wobei der Blanchierungsschritt beinhaltet, das Lebensmittelprodukt einem aktiven Blanchierungsmedium unter Blanchierungsbedingungen in einer Blanchierungssektion auszusetzen, um verbrauchtes Blanchierungsmedium herzustellen, Entziehen reduzierender Zucker und/oder Asparagine von dem verbrauchten Blanchierungsmedium, um aktives Blanchierungsmedium zu produzieren unter Benutzung von Entzuckerungs- und/oder Asparagin entziehenden Mittel und Wiederbenutzung des aktiven Blanchierungsmediums, unter der Bedingung, dass falls nur einer der reduzierenden Zucker und Asparagine dem verbrauchten Blanchierungsmedium entzogen wird und das besagte Lebensmittelprodukt Kartoffelscheiben sind, die besagten reduzierenden Zucker oder Asparagine nicht unter Verwendung einer Kolonne entzogen wird, die immobilisierte Enzyme enthält, wobei der Abfluss von der Kolonne zu den Kartoffelscheiben zurückgeführt wird.

Schließlich sei noch auf die US 3,362,836 A hingewiesen, welche ein Verfahren zur Herstellung von Albumen (Nährgewebe, Eiweiß) offenbart.

M.G. Aziz et al. beschreibt die Biotransformation von Zucker aus Ananassaft in diätetische Derivate unter Verwendung einer zellfreien Oxidoreduktase aus Zymomonas mobilis zusammen mit kommerzieller Invertase.

CH 668 887 beschreibt das Fruchsaft, z.B. Apfelsaft durch biologischen Abbau von einem mindestens überwiegenden Teil seines nativen Zuckers befreit wird, indem man den zuckerhaltigen Fruchtsaft in kontinuierlicher oder halbkontinuierlicher Kultur mit einer Hefe unter Kulturbedingungen behandelt, unter welchen die Hefe einen mindestens überwiegenden Teil des im Fruchtsaft enthaltenen Zuckers praktisch ohne Ethanolbildung bis zur Kohlendioxid/Wasser-Stufe abbaut; durch Abtrennen der Biomasse wird ein alkoholfreier Fruchtsaft mit praktisch auf Null vermindertem Zuckergehalt gewonnen.

Darüber hinaus beschränken sich bestehende Verfahren zum Erhalt zuckerreduzierter Fruchtprodukte meist auf die Verdünnung der Zuckerstoffe mit Wasser. Damit verringert sich aber auch die Konzentration der in den Früchten bzw. daraus erhaltenen Fruchtedukten natürlicherweise enthaltenen physiologisch wichtigen sekundären Inhaltsstoffe (insb. Polyphenole, Vitamine, etc.). Die sich damit ebenfalls verschiebenden Geschmacksnoten werden in bisher entwickelten Fruchtprodukten vermehrt durch Einsatz unerwünschter Aromastoffe und Farbstoffe ausgeglichen, die dem immer stärker werdenden Wunsch des Verbrauchers nach Lebensmitteln mit natürlichen Inhaltsstoffen natürlich nicht gerecht werden können.

Als wesentliches Problem zeigt sich bei der Anwendung des bisherigen Stands der Technik die Bildung von zuckerreduzierten Fruchtprodukten, die aufgrund ihres durch den fehlenden Zucker unausgewogenen Verhältnis der im zuckerreduzierten Fruchtprodukt enthaltenen Säuren und Zuckerstoffe, im Kontext der vorliegenden Erfindung kurz als "Zucker-Säure-Verhältnis" bezeichnet, bislang zu keinen geschmacks-sensorisch akzeptablen zuckerreduzierten Fruchtprodukten führen.

### Der Erfindung zugrundeliegende Aufgabe

Hiervon ausgehend liegt der vorliegenden Erfindung die Aufgabe zugrunde, ein Verfahren zur biotechnologischen Reduzierung von Zuckerstoffen in Fruchtedukten zwecks Erhalts zuckerreduzierter Fruchtprodukte bereitzustellen, welches sowohl die in den Fruchtedukten enthaltenen physiologisch wichtigen sekundären Inhaltsstoffe erhält als auch gleichzeitig den oft unerwünscht hohen Zuckergehalt stark reduziert und ausgewogene Geschmacksnoten ohne Einsatz unerwünschter Aroma- und Farbstoffe beibehält. Um eine Zerstörung oder Schädigung der gesundheitlich bedeutenden natürlichen Inhaltsstoffe der Fruchtedukte (insb. Polyphenole, Vitamine, Säuren) zu vermeiden sollen darüber hinaus insbesondere für die angesprochene Reduzierung von Zuckerstoffen in Fruchtedukten nur Verfahren zum Einsatz kommen, die bei produktschonenden Temperaturen ablaufen können und ferner keine extremen pH-Werte (insb. nicht größer 6 pH und nicht kleiner 3 pH) oder den Zusatz organischer Lösungsmittel oder den Zusatz von Süßstoffen benötigen.

### Erfindungsgemäße Lösung

Diese Aufgabe wird durch ein Verfahren nebst Vorrichtung zur biotechnologischen Reduzierung von Zuckerstoffen in Fruchtedukten zwecks Erhalts zuckerreduzierter Fruchtprodukte mit den Merkmalen der unabhängigen Patentansprüche gelöst.

Das erfindungsgemäße Verfahren zur biotechnologischen Reduzierung von Zuckerstoffen in Fruchtedukten zwecks Erhalts zuckerreduzierter Fruchtprodukte zeichnet sich durch enzymatische Reaktionsprozesse aus. Es ist gekennzeichnet durch einen Regelprozess, mit dem der pH-Wert im zuckerreduzierten Fruchtprodukt so auf einen vorgegebenen höheren Wert gegenüber dem pH-Wert im Fruchtedukt eingeregelt wird,
- dass bei einer Reduzierung der Zuckerstoffe um mindestens 30 Gew-% bis kleiner 40 Gew-% der pH-Wert zwischen 0,6 und 1,0 pH-Einheiten erhöht ist; oder
- dass bei einer Reduzierung der Zuckerstoffe um mindestens 40 Gew-% bis kleiner 50 Gew-% der pH-Wert zwischen 0,7 und 1,1 pH-Einheiten erhöht ist; oder
- dass bei einer Reduzierung der Zuckerstoffe um mindestens 50 Gew-% bis kleiner 65 Gew-% der pH-Wert zwischen 0,8 und 1,2 pH-Einheiten erhöht ist; oder
- dass bei einer Reduzierung der Zuckerstoffe um mindestens 65 Gew-% bis kleiner 80 Gew-% der pH Wert zwischen 0,9 und 1,3 pH Einheiten erhöht ist; oder
- dass bei einer Reduzierung der Zuckerstoffe um mindestens 80 Gew-% der pH-Wert zwischen 1,0 und 1,4 pH-Einheiten erhöht ist;
wobei die zuckerreduzierten Fruchtprodukte Ganzfruchtgetränke oder Obst- und/oder Gemüsesafte sind,
(a) gekennzeichnet durch eine
   - enzymatische Umwandlung von Saccharose in Glucose und Fructose mittels Invertase; und/oder
   - enzymatische Umwandlung von Fructose zu Glucose mittels Glucose- Isomerase und
(b) gekennzeichnet durch einen
   - enzymatischen Abbau von Glucose unter Bildung von Gluconsäure und Wasserstoffperoxid mittels Glucose-Oxidase
   - bei gleichzeitigem Einsatz von Katalase, um entstandenes Wasserstoffperoxid in Wasser und Sauerstoff umzuwandeln.

Das erfindungsgemäß vorgesehene Einregeln eines höheren pH-Wertes im zuckerreduzierten Fruchtprodukt gegenüber dem pH-Wert im Fruchtedukt hat eine Verhinderung der geschmacklichen Übersäuerung zum Vorteil und zwar unabhängig davon, ob alternativ oder kumulativ enzymatische und/oder fermentative Reaktionsprozesse Anwendung fanden. Vielmehr wurde erstmals ein zuckerreduziertes Fruchtprodukt mit geschmacks-sensorisch ausgewogenem Säure-Zucker-Verhältnis, also einem ausgewogenes Verhältnis saurer und süßer Geschmacksnoten, erhältlich, ohne dass es dem sonst üblichen Zusatz unerwünschter Aroma- und Farbstoffe bedurfte.

### Bevorzugte Ausgestaltungen der Erfindung

Vorteilhafte Ausgestaltungen und Weiterbildungen, welche einzeln oder in Kombination miteinander einsetzbar sind, sind Gegenstand der abhängigen Ansprüche.

Es ist hierin beschrieben, dass bei fermentativ gebildeten Zuckeralkoholen der niedriger ausfallende pH-Wert zum Gew-%-Anteil der Zuckeralkohole dergestalt linear korreliert, dass beispielsweise
- bei 1,0 Gew-% fermentativ gebildeter Zuckeralkohole die pH-Wert-Anhebung um 0,1 pH-Einheiten, und
- bei 2,0 Gew-% fermentativ gebildeter Zuckeralkohole die pH-Wert-Anhebung um 0,2 pH-Einheiten, und
- bei 3,0 Gew-% fermentativ gebildeter Zuckeralkohole die pH-Wert-Anhebung um 0,3 pH-Einheiten
geringer ausfällt; was vorteilhaft die Ausgestaltung des Regelprozesses bei kombinierten Reaktionsprozessen vereinfacht.

Das erfindungsgemäße Verfahren ist gekennzeichnet durch eine enzymatische Umwandlung von Saccharose in Glucose und Fructose mittels Invertase und/oder durch eine enzymatische Umwandlung von Fructose zu Glucose mittels Glucose-Isomerase.

Saccharose, auch als Haushalts- oder Kristallzucker bezeichnet, ist aufgebaut aus einem α-D-Glucose und einem β-D-Fructose Molekül, die über eine α,β-(1-2)-glycosidische Bindung miteinander verknüpft sind. In Gegenwart von Wasser, was in Früchten bzw. daraus erhaltenen Fruchtedukten neben den Zuckerstoffen den wichtigsten Inhaltsstoff ausmacht, kann in dieser Ausgestaltung mit Hilfe des Enzyms Invertase (β-D-Fructofuranosidase) die Saccharose vorteilhaft in ein Fructose-(Fruchtzucker)- und ein Glucose-(Traubenzucker)-Molekül aufgespalten werden. Das Reaktionsschema dieser Hydrolyse von Saccharose in Glucose und Fructose mittels Invertase lässt sich wie folgt darstellen:

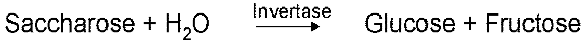

Somit kann durch Einsatz des Enzyms Invertase die im Fruchtedukt vorhandene Saccharose soweit gewünscht auch vollständig in Glucose und Fructose umgewandelt und mittels enzymatischer und/oder fermentativer Reaktionsprozesse vorteilhaft weiter umgewandelt und/oder abgebaut werden.

Durch den alternativen oder kumulativen Einsatz des Enzyms Glucose-Isomerase kann im Fruchtedukt vorhandene Fructose vorteilhaft in Glucose umgewandelt und mittels enzymatischer und/oder fermentativer Reaktionsprozesse abgebaut und/oder weiter umgewandelt werden.

Ferner ist die erfindungsgemäße Ausgestaltung des Verfahrens gekennzeichnet durch einen enzymatischen Abbau von mittels Invertase und/oder Glucose-Isomerase ggf. gebildeter und/oder in dem Fruchtedukt bereits vorhandener Glucose mittels des Enzyms Glucose-Oxidase unter Bildung von Gluconsäure. Allerdings entsteht dabei als unerwünschtes Nebenprodukt Wasserstoffperoxid (H₂O₂). Dieses Wasserstoffperoxid kann aufgrund seiner stark oxidativen Wirkung die im Fruchtedukt wichtigen sekundären Pflanzeninhaltsstoffe zerstören.

Um dies zu vermeiden wird zeitgleich das Enzym Katalase eingesetzt , welches entstehendes Wasserstoffperoxid zu unschädlichem Wasser und Sauerstoff umwandelt. Das Reaktionsschema des Glucose-Oxidase/Katalase-Enzymsystems lässt sich wie folgt darstellen:

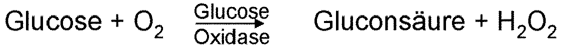

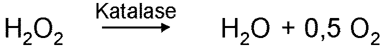

Da mit dem Einsatz der Enzymkombination aus Glucose-Oxidase und Katalase gleichzeitig mit der Umsetzung eines Glucose Moleküls ein halbes Sauerstoffmolekül aus dem Fruchtedukt entfernt wird, führt der Glucoseabbau vorteilhaft auch zu einer Entfernung von Sauerstoff und somit zu einem Schutz insbesondere oxidationsempfindlicher Vitamine und Polyphenole im Fruchtedukt.

Allerdings können die aufgeführten Enzymreaktionen aus Glucose-Oxidase und Katalase bei Abbau des angesprochenen Zuckerstoffes in dem Fruchtedukt aufgrund eines schnellen pH-Abfalls und der daraus folgenden Inaktivierung der eingesetzten Enzyme recht schnell enden. Dann würde zusätzlich die Reaktion zur Bildung der Gluconsäure durch den schnellen Verbrauch der im Ausgangsprodukt vorhandenen Sauerstoffmengen unwirtschaftlich langsam ablaufen.

Daher ist gemäß einer Weiterbildung des Verfahrens bevorzugt, während der enzymatischen Reaktionen mittels Glucose-Oxidase und Katalase für den Glucoseabbau benötigter Sauerstoff zuzuführen. Insbesondere ist bevorzugt, dass während der enzymatischen Reaktionsprozesse mittels Glucose-Oxidase und Katalase die Sauerstoffzufuhr auf Sättigungen zwischen 5 % und 60 %, vorzugsweise zwischen 10 % und 40 %, besonders bevorzugt zwischen 30 % und unter 40 %, eingeregelt wird. Insbesondere konnte bei Einstellungen des Sauerstoffgehalts auf vorzugsweise unter 40 % eine unerwünschte Oxidation der ernährungsphysiologisch wertvollen Inhaltsstoffe im Fruchtedukt gewährleistet werden. In dem allein der für den Abbau des Zuckerstoffes Glucose benötigte Sauerstoff für die Lebensfähig- und Reaktionsfreudigkeit der Enzyme zur Verfügung gestellt wird, ist vorteilhaft ein mit wirtschaftlicher Prozessgeschwindigkeit durchführbares Verfahren zur biotechnologischen Reduzierung oder gar Eliminierung des Zuckerstoffes Glucose in beliebiger oder gar 100 %-iger Höhe seiner ursprünglich natürlichen und ggf. auch enzymatisch umgewandelten Mengen, insbesondere aber ein kommerziell interessanter Glucoseabbau bei entsprechender Kalorienreduzierung von mindestens 30 % in Fruchtedukten zwecks Erhalts zuckerreduzierter Fruchtprodukte, bereitgestellt. Zweckmäßiger Weise wird Sauerstoff gleichmäßig in eine zur Durchführung des Verfahrens ausgelegte Vorrichtung eingeblasen, was die Konstant-Haltung angestrebter Sättigungswerte zum Vorteil hat.

Kumulativ zu enzymatischen Reaktionsprozessen, insbesondere mittels Glucose-Oxidase und Katalase, ist eine erfindungsgemäß bevorzugte Weiterbildung des Verfahrens gekennzeichnet durch einen fermentativen Abbau von Fructose und/oder Glucose unter Bildung von süßenden Zuckeralkoholen mittels spezieller Mikroorganismen wie Leuconostoc mesenteroides, Monitiella tomentasa, Candida magnoliae. Das fermentative Verfahren kann unter aeroben oder bevorzugt, dann aber nicht zeitgleich zu einer eingeregelten Sauerstoffzufuhr, unter anaeroben Bedingungen durchgeführt werden.

Im Rahmen der vorliegenden Erfindung finden vorzugsweise solche Mikroorganismen Verwendung, die - trotz eines niedrigen pH-Werts und kaum vorhandener Stickstoffquellen (die normalerweise Grundlage für das Wachstum von Mikroorganismen ist) - im Fruchtedukt und in Gegenwart fruchteigener Bakteriozide (wie z.B. Polyphenole) wachsen, insbesondere Pilze, Hefen und/oder Bakterien.

Diesbezüglich positive Erfahrungen konnten beispielsweise mit dem Milchsäurebakterium *Leuconostoc mesenteroides* gewonnen werden. Allerdings bildet dieses laxierend wirkendes Mannit als Zuckeralkohol.

Besonders bevorzugt sind daher Mikroorganismen, die laxiere-freie, also nicht abführend wirkende, süßende Zuckeralkohole wie insbesondere Erythritol bilden. Diesbezüglich positive Erfahrungen konnten beispielsweise mit dem Pilz *Monitiella tomentosa* oder mit der Hefe *Candida magnoliae* gewonnen werden.

Der Einsatz von Pilzen, Hefen und/oder Bakterien bewirkt vorteilhaft, dass von einer beliebigen Zuckermenge (= 100%) bereits etwa die Hälfte (= 50 %) von den Mikroorganismen "verbraucht" werden und die anderen 50 % zu süßenden, kalorienreduzierenden Verbindungen im Verhältnis von etwa 2 : 1 umgewandelt werden, was bezogen auf die eingesetzte Zuckermenge (= 100 %) eine effektive Zucker- bzw. Kalorienreduzierung von ursprünglich 100 % um 75 % auf 25 % im angestrebten Fruchtprodukt zum Vorteil hat.

Ein Verfahren zur anaeroben Fermentation, insbesondere als Weiterbildung des erfindungsgemäßen Verfahrens, zeichnet sich dadurch aus, dass vor und/oder während einem Einsatz anaerober Mikroorganismen die Entfernung von Sauerstoff vollständig enzymatisch mittels der Reaktionsprozesse Glucose-Oxidase- und Katalase-Reaktionen erfolgt.

Die vollständige Entfernung des Sauerstoffs mittels der angesprochenen enzymatischen Reaktionsprozesse Glucose-Oxidase- und Katalase-Reaktionen ermöglicht vorteilhaft die Herstellung anaerober Reaktionsbedingungen ohne hohen apparativen und teuren Aufwand, wie dies im Stand der Technik zum Wachstum der Fructose und/oder Glucose abbauenden und süße, nicht kariogenen und Kalorien reduzierende Zuckeralkohole bildende Mikroorganismen bislang notwendig ist.

In einer Weiterbildung der vorliegenden Erfindung erfolgt das Einregeln auf einen höheren pH-Wert, vorzugsweise automatisiert, während der Reaktionsprozesse.

Das, vorzugsweise automatisierte, Einregeln auf einen höheren pH-Wert bereits während der Reaktionsprozesse hat eine zeitliche Beschleunigung der Reaktionsprozesse und damit erhöhte Wirtschaftlichkeit zum Vorteil, da die erfindungsgemäß eingesetzten Enzyme und Mikroorganismen bei eher neutralen oder weniger sauren pH-Werten, insbesondere aber bei höheren pH-Werten als im Fruchtedukt anzutreffen, schneller reagieren.

Allerdings ist es wichtig, den pH Wert in den Fruchtedukten bzw. -produkten vor, während und nach der biotechnologischen Reaktion nicht über 6,0 einzustellen, da es oberhalb dieses pH Werts zur irreversiblen Zerstörung ernährungsphysiologisch wichtiger sekundärer Pflanzeninhaltstoffe wie Polyphenole, Anthocyane oder auch von natürlichen Aromastoffen kommen kann.

Erfindungsgemäß bevorzugt erfolgt das Einregeln auf einen höheren pH-Wert durch Zugabe geschmacksneutraler Salze und/oder Salzsuspensionen, wie insbesondere Magnesiumoxid und/oder Magnesiumoxidsuspension.

Die angesprochenen Magnesiumverbindungen haben zum Vorteil, dass diese geschmacklich besser deutlich sind als ebenfalls denkbare Kalium-, Calcium oder Natriumverbindungen: Natriumverbindungen schmecken salzig; Kalium- und Calciumverbindungen bitter.

Ferner wird hierin auch eine Vorrichtung zur Durchführung eines Verfahrens wie zuvor beschrieben, welche dadurch gekennzeichnet ist, dass in dieser zumindest
- Mittel zum gleichmäßigen Eintrag von Sauerstoff auf einen für die empfindlichen sekundären Inhaltsstoffe im Fruchtedukt verträglichen Gehalt, und/oder
- Mittel zum Einregeln eines pH-Werts durch Zudosierung eines Säureregulators auf einen höheren Wert gegenüber dem pH-Wert im Fruchtedukt;
vorgesehen sind.

In einer Ausgestaltung der Vorrichtung zur Durchführung des erfindungsgemäßen Verfahrens ist diese bevorzugt geschlossen ausgebildet und arbeitet diese bevorzugt bei Raumtemperatur.

Dabei hat eine bevorzugt geschlossen ausgebildete Vorrichtung bei enzymatischen Reaktionsprozessen eine Vermeidung von Verlusten (Verflüchtigungen) des zugegebenen Sauerstoffs sowie der Aromen im Fruchtedukt und bei (zeitlich nachgelagerten anaeroben) fermentativen Reaktionsprozessen die Gewährleistung der Beibehaltung anaerober Bedingungen zum Vorteil.

Ebenfalls ist eine Vorrichtung zur Durchführung des erfindungsgemäßen Verfahrens vergleichsweise preiswert erhältlich, wenn sowohl die enzymatischen als auch die fermentativen Reaktionsprozesse bevorzugt bei produktschonenden Temperaturen, insbesondere bei Raumtemperatur, erfolgen.

Mittels der vorliegenden Erfindung sind zuckerreduzierte Fruchtprodukte erhältlich wie Ganzfruchtgetränke (Smoothies) oder Obst- und/oder Gemüsesäfte (gleich ob unverdünnt als Direktsaft oder rückverdünnt als Fruchtsaft aus Fruchtsaftkonzentrat abgefüllt) ; wobei der pH-Wert im zuckerreduzierten Fruchtprodukt erfindungsgemäß auf einen vorgegebenen höheren Wert gegenüber dem pH-Wert im Fruchtedukt eingeregelt ist.

Die Herstellung zuckerreduzierter Fruchtprodukte durch das hier vorgestellte biotechnologische Verfahren bietet eine Möglichkeit, sich mit innovativen Fruchtprodukten über ein Alleinstellungsmerkmal auf dem Markt zu etablieren. Momentan gibt es nämlich kein Fruchtprodukt, das auf der einen Seite die in Früchten enthaltenen gesunden natürlichen Inhaltsstoffe beinhaltet, gleichzeitig stark zuckerreduziert ist und zudem ein geschmacks-sensorisch ausgewogenes Säure-Zucker-Verhältnis aufweist.

### Kurzbeschreibung der Zeichnungen

Zusätzliche Einzelheiten und weitere Vorteile der Erfindung werden nachfolgend an Hand bevorzugter, durch Laborversuch belegte Ausführungsbeispiele (nachfolgend mit AB abgekürzt), auf welche die vorliegende Erfindung jedoch nicht beschränkt ist, und in Verbindung mit der beigefügten Zeichnung beschrieben.

Es zeigen schematisch:
- Fig. 1: in einem Prozessdiagramm wesentliche Schritte eines erfindungsgemäßen Verfahrens zur biotechnologischen Reduzierung von Zuckerstoffen in Fruchtedukten zwecks Erhalts zuckerreduzierter Fruchtprodukte durch enzymatische und/oder fermentative Reaktionsprozesse;
- Fig. 2: ein Reaktionsschema enzymatischer Reaktionsprozesse;
- Fig. 3: einen Verlauf eines eingeregelten pH-Wertes bei 4,3;
- Fig. 4: eine eingeregelte Sauerstoffsättigung bei im Mittel 30 % Sauerstoff O₂;
- Fig. 5: einen zeitlichen Verlauf des Glucoseabbau in einem Traubensaft mittels Glucose-Oxidase;
- Fig. 6: eine Entfernung des Zuckerstoffes Saccharose in einem Orangensaft mittels des Enzyms Invertase;
- Fig. 7: eine Gegenüberstellung der Zuckerstoff-Gehalte verschiedener Direktsäfte vor und nach einem enzymatischen Zuckerabbau;
- Fig. 8: die Abhängigkeit eines ausgeglichenen Säure-Zucker-Verhältnisses verschiedener Fruchtsäfte nach einer enzymatischen Zuckerreduzierung vom pH-Wert;
- Fig. 9: ein Reaktionsschema fermentativer Reaktionsprozesse;
- Fig. 10: eine fermentative Reduzierung der Zuckerstoffe Glucose (links) und Fructose (rechts) mittels des Pilzes *Moniliella tomentosa* unter Bildung von Erythritol;
- Fig. 11: ein Reaktionsschema enzymatisch und fermentativ kombinierter Reaktionsprozesse mit einem fermentativem Schwerpunkt;
- Fig. 12: ein Reaktionsschema der Kombination aller zuvor beschriebenen enzymatischen und fermentativen Reaktionsprozesse mit beliebig wählbaren Schwerpunkten;
- Fig. 13: einen apparativen Labor-Aufbau;
- Fig. 14: eine Sauerstoffsättigung im Verlauf eines enzymatischen Abbaus der Zuckerstoffe Saccharose und Glucose in einem Ananas Direktsaft;
- Fig. 15: einen pH Verlauf während des enzymatischen Abbaus der Zuckerstoffe Saccharose und Glucose in einem Ananas Direktsaft; und
- Fig. 16: die prinzipielle Veränderung der Konzentration der Zuckerstoffe (Saccharose und Glucose) während des enzymatischen Abbaus und der Bildung von Zuckeralkohol während des fermentativen Abbaus von Fructose in einem Ananas Direktsaft.

### Ausführliche Figurenbeschreibung

Zuckerstoffe bilden neben Wasser die wichtigsten Inhaltsstoffe von Früchten. Die Zuckerstoffe setzen sich dabei in erster Linie aus der Saccharose, der Glucose und der Fructose zusammen, wobei sich deren prozentualen Gesamtmasse-Anteile je nach Frucht stark unterscheiden können (vgl. die in der Beschreibungseinleitung niedergelegte Tabelle).

Diese Zuckerstoffe finden sich dann auch zum überwiegenden Teil in den aus Fruchtedukten produzierten Fruchtprodukten wieder. Eine Untersuchung verschiedener kommerziell erhältlicher Direktsäfte beispielsweise, wobei unter Direktsäfte solche Obst- und/oder Gemüsesäfte zusammengefasst werden, die direkt nach der Saftpressung ohne zusätzliche Zuckerzugabe abgefüllt und nur die Zuckerstoffe aus dem jeweiligen Fruchtedukt enthalten, ergibt die folgenden typischen Gehalte an Zuckerstoffen:

| **Direkt-Saft** | **Saccharose [g/l]** | **Glucose [g/l]** | **Fructose [g/l]** |
|---|---|---|---|
| | | | |
| Ananassaft | 41,7 | 32,4 | 35,8 |
| Grapefruit | 14,4 | 35,4 | 39,4 |
| Orangensaft | 31,7 | 27,8 | 32,3 |
| Traubensaft | n.n. | 86,8 | 87,6 |
| | | | |

Es zeigt sich, dass vor allem in den Traubensäften hohe Gehalte an Glucose und Fructose befinden. Saccharose dagegen findet sich vor allem in Ananas- und Orangensaft in höherer Konzentration.

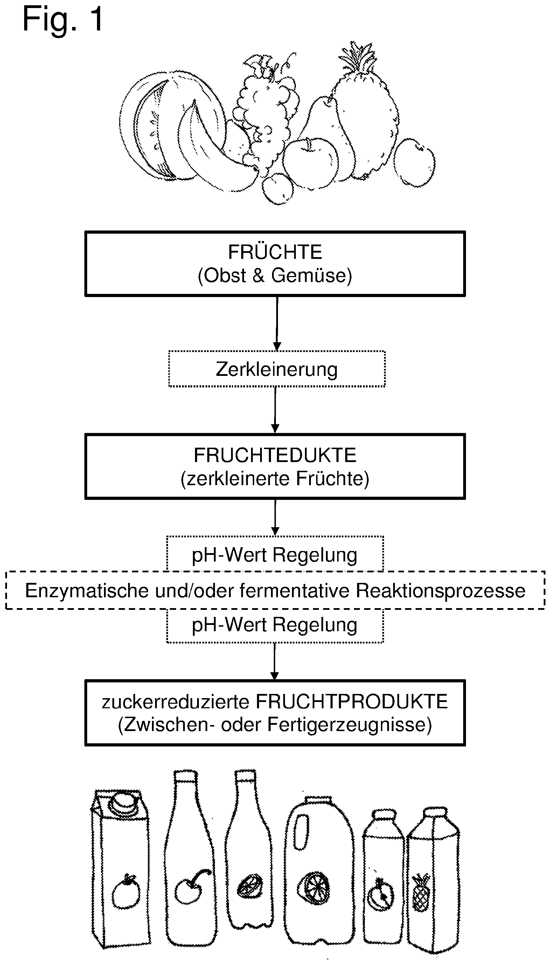

**Fig. 1** veranschaulicht an Hand eines schematischen Prozessdiagramms wesentliche Schritte eines erfindungsgemäßen Verfahrens zur biotechnologischen Reduzierung von Zuckerstoffen in Fruchtedukten zwecks Erhalts zuckerreduzierter Fruchtprodukte durch enzymatische und/oder fermentative Reaktionsprozesse. Wie in Fig. 1 dargestellt zeichnet sich das erfindungsgemäße Verfahren durch einen Regelprozess aus, mit dem der pH-Wert im zuckerreduzierten Fruchtprodukt auf einen vorgegebenen höheren Wert gegenüber dem pH-Wert im Fruchtedukt eingeregelt wird. Das erfindungsgemäß vorgesehene Einregeln des pH-Wertes im zuckerreduzierten Fruchtprodukt auf einen vorgegebenen höheren Wert gegenüber dem pH-Wert im Fruchtedukt hat eine Verhinderung der geschmacklichen Übersäuerung im zuckerreduzierten Fruchtprodukt zum Vorteil und zwar unabhängig davon, ob alternativ oder kumulativ enzymatische und/oder fermentative Reaktionsprozesse Anwendung fanden:

### AB 1: Etablierung enzymatischer Verfahrens zur Entfernung von Glucose, Saccharose und/oder Fructose

Aufgrund des hohen Brennwerts und der kariogenen Wirkung der Zuckerstoffe Glucose, Saccharose und/oder Fructose können im Rahmen der vorliegenden Erfindung enzymatische Reaktionsprozesse vorgesehen sein, mittels welchen Saccharose, Fructose und/oder insbesondere Glucose in den Früchten bzw. Fruchtedukten enzymatisch abgebaut werden.

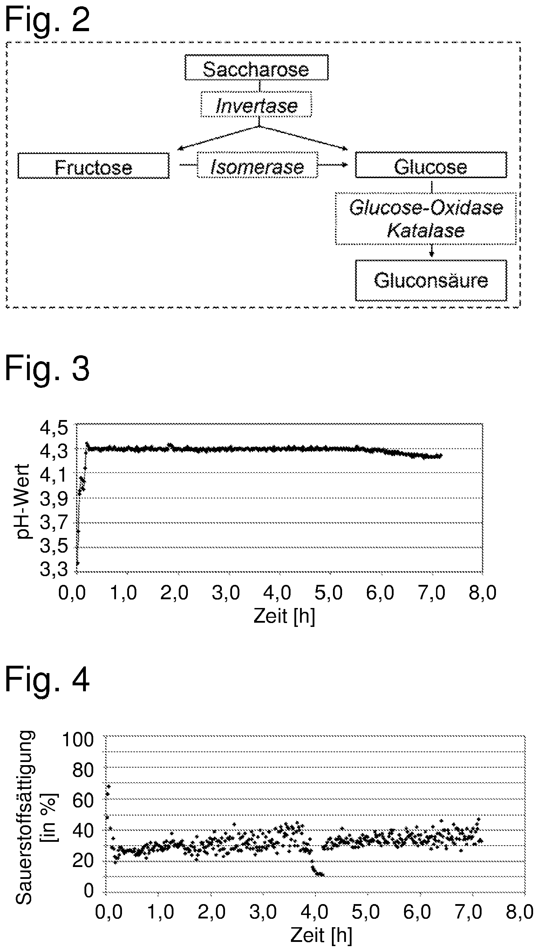

**Fig. 2** zeigt ein Reaktionsschema enzymatischer Reaktionsprozesse. Erkennbar ist, wie mit einer Kombination der Enzyme Invertase, Glucose-Oxidase und Katalase sowohl der in Fruchtedukten vorhandene Zuckerstoff Saccharose, als auch der dabei gebildete Zuckerstoff Glucose entfernt werden kann. Mittels des Enzyms Isomerase kann sogar auch der Zuckerstoff Fructose in Glucose umgewandelt und enzymatisch abgebaut werden.

### AB 1.1: Glucose-Abbau mittels Glucose-Oxidase

Dabei konnten unwirtschaftlich langsamen Reaktionsprozessen mit einem Regelprozess zur Regelung des pH-Wertes auch während der Reaktionsprozesse und/oder einem Regelprozess zur Zufuhr benötigten Sauerstoffes entgegengewirkt werden, wobei der Sauerstoff bevorzugt gleichmäßig in eine Vorrichtung zur Durchführung des Verfahrens eingeblasen wurde und dabei die Sauerstoffzufuhr vorzugsweise auf Sauerstoffsättigungen zwischen 5 % und 60 %, insbesondere zwischen 10 % und 40 %, besonders bevorzugt zwischen 30 % und unter 40 %, eingeregelt wurde.

**Fig. 3** zeigt den Verlauf des pH-Wertes einer, bevorzugt automatischen, Regelung während der Reaktionsprozesse bei beispielhaft pH 4,3. Erkennbar ist, wie die bevorzugt automatische pH-Regelung den pH-Wert während der Reaktionsprozesse zuverlässig in einem Bereich von +/- 0,05 pH Einheiten hält.

**Fig. 4** zeigt die automatisch eingeregelte Sauerstoffsättigung in einem Direktsaft beispielsweise auf im Mittel 30 % Sauerstoff O₂. Aufgrund der hohen Empfindlichkeit der Sauerstoffelektrode und der Gasblasen im Fruchtedukt mögen hier zwar kurzfristige Schwankungen im Sauerstoffgehalt der Lösung beobachtet werden; einen negativen Einfluss auf die Qualität des zuckerreduzierten Fruchtproduktes konnte aber nicht festgestellt werden.

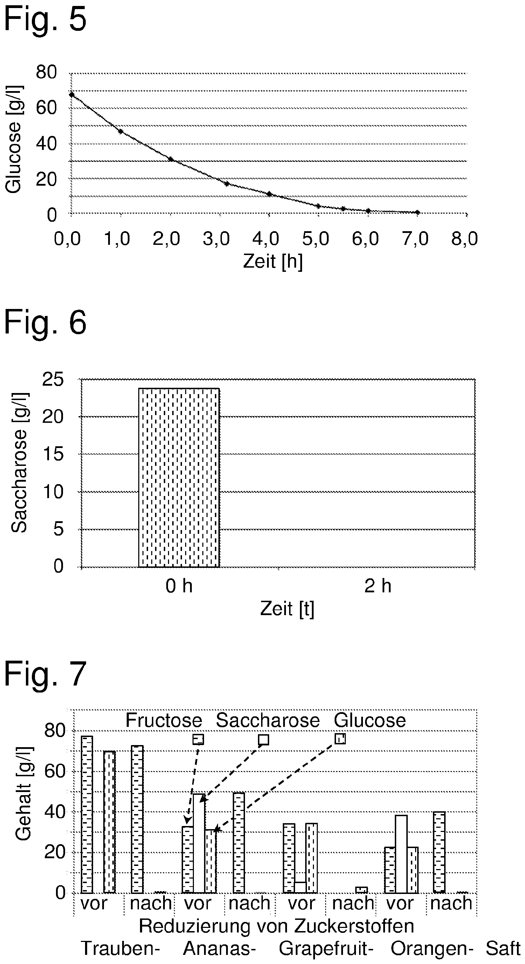

**Fig. 5** zeigt den zeitlichen Verlauf des Abbaus von Glucose in einem Traubensaft mittels Glucose-Oxidase. Eindrucksvoll ist verdeutlicht, wie mit den zuvor beschriebenen Regelprozessen (Regelung 4,3 pH, 30 % Sauerstoff) innerhalb von nur 7 h möglich wurde, die komplette Glucose eines Traubensafts mittels der Enzyme Glucose-Oxidase und Katalase vollständig abzubauen. Analoge Versuche mit Himbeer-, Kirsch-, Orangen-, Ananas und Grapefruitsaft führten zu vergleichbaren Ergebnissen und zeigen die universelle Übertragbarkeit und Wirtschaftlichkeit des entwickelten Verfahrens auf die meisten relevanten Fruchtmatrizes.

### AB 1.2: Saccharose-Entfernung mittels Invertase

Auch der Zuckerstoff Saccharose findet sich, mit Ausnahme der Traube, in den meisten Obstsorten mit teils signifikantem Anteil. Neben dem Glucose-Abbau wird daher auch ein ergänzendes Verfahrens zur Entfernung auch dieses Zuckerstoffes bereitgestellt. Insbesondere wird vorgeschlagen, mittels des Enzyms Invertase (Saccharase) die Saccharose zu gleichen Teilen in Glucose und Fructose aufzuspalten. Da die in verschiedenen Versuchen eingesetzte kommerziell erhältliche Saccharase (Invertase) eine sehr hohe Enzymaktivität aufweist, gelang es erstaunlicherweise, in einem Orangensaft einen Saccharosegehalt von ca. 24 g/l (Orangensaft) innerhalb von nur 2 h vollständig zu Glucose und Fructose abzubauen:
**Fig. 6** zeigt die Entfernung des Zuckerstoffes Saccharose in einem Orangensaft mittels des Enzyms Invertase, vornehmlich bei Raumtemperaturen insbesondere zwischen 20 °C und 30 °C, und einem während der Reaktionsprozesse eingeregelten pH-Wert von 4,3.

### AB 1.3: Glucose- und Saccharose-Abbau in der Fruchtmatrix

In den im Folgenden dargestellten Versuchen konnten auch die vorteilhaften Möglichkeiten und Auswirklungen einer Kombination der drei gezeigten Enzymreaktionen zur vollständigen Entfernung insbesondere der Zuckerstoffe Saccharose und Glucose aus der Fruchtedukte-Matrix untersucht werden.

Dazu wurden verschiedenen Ostsäften eine Mischung der drei Enzyme Invertase, Glucose-Oxidase und Katalase zugegeben und anschließend während der Reaktionsprozesse unter Regelung auf einen gegenüber dem pH-Wert im Fruchtedukt vorgegebenen höheren pH-Wert und unter Regelung des Sauerstoffgehalts im aufgebauten Reaktor die Zuckergehalte vor und nach Abschluss des enzymatischen Abbaus (innerhalb von 7 Stunden) quantifiziert.

**Fig. 7** zeigt eine Gegenüberstellung der Zuckerstoff-Gehalte verschiedener Direktsäfte vor und nach einem enzymatischen Zuckerabbau. Die Ergebnisse mit verschiedenen Direktsäften ist insoweit eindrucksvoll, demnach die vollständige Entfernung der Zuckerstoffe Glucose und Saccharose mit Enzymen in der Fruchtedukte-Matrix innerhalb von 7 Stunden selbst in einem Traubensaft mit einem Glucosegehalt von über 70 g/l vollständig gelang.

### AB 1.4: Fructose-Entfernung mittels Glucose-Isomerase

Bereits die vorstehenden Ergebnisse weisen die Wirksamkeit der entwickelten enzymatischen Reaktionsprozesse in einem Verfahren zur vollständigen Entfernung der Zuckerstoffe Glucose und der Saccharose aus verschiedenen Fruchtedukte-Matrizes eindrucksvoll nach. Diese Reaktionsprozesse können je nach Bedarf noch um die eingangs gezeigte Glucose-Isomerase zur Umwandlung von Fructose erweitert werden.

Dabei ist zu beachten, dass die Gleichgewichtslage der Glucose-Isomerase sich meist bei einem Verhältnis von 1:1 (Glucosegehalt : Fructosegehalt) einstellt, weshalb erfindungsgemäß bevorzugt erst der zusätzliche Einsatz der Glucose-Oxidase eine Entfernung der Glucose aus diesem Gleichgewicht und damit einen vollständigen Abbau der Fructose über Glucose (Glucose Isomerase) zu Gluconsäure (Glucose-Oxidase) ermöglicht.

### AB 1.5: Abhängigkeit ausgeglichener Säure-Zucker Verhältnisse

Des Weiteren wurde überraschend festgestellt, dass das Einregeln eines pH-Wertes nicht nur während enzymatischer und/oder fermentativer (dazu später) Reaktionsprozesse sondem im zuckerreduzierten Fruchtprodukt selbst so auf einen vorgegebenen höheren Wert gegenüber dem pH-Wert im Fruchtedukt,
- dass bei einer Reduzierung der Zuckerstoffe um mindestens 30 Gew-% bis kleiner 40 Gew-% der pH-Wert zwischen 0,6 und 1,0 pH-Einheiten erhöht ist; oder
- dass bei einer Reduzierung der Zuckerstoffe um mindestens 40 Gew-% bis kleiner 50 Gew-% der pH-Wert zwischen 0,7 und 1,1 pH-Einheiten erhöht ist; oder
- dass bei einer Reduzierung der Zuckerstoffe um mindestens 50 Gew-% bis kleiner 65 Gew-% der pH-Wert zwischen 0,8 und 1,2 pH-Einheiten erhöht ist; oder
- dass bei einer Reduzierung der Zuckerstoffe um mindestens 65 Gew-% bis kleiner 80 Gew-% der pH Wert zwischen 0,9 und 1,3 pH Einheiten erhöht ist; oder
- dass bei einer Reduzierung der Zuckerstoffe um mindestens 80 Gew-% der pH-Wert zwischen 1,0 und 1,4 pH-Einheiten erhöht ist;
eine Verhinderung der geschmacklichen Übersäuerung zum Vorteil hat, und zwar unabhängig davon, ob alternativ oder kumulativ enzymatische und/oder fermentative (dazu später) Reaktionsprozesse Anwendung fanden,
wobei die vorgenannten pH-Werte jeweils auch um bis zu 0,1 oder bis zu 0,2 pH-Einheiten höher oder niedriger ausfallen können; und/oder
wobei bei fermentativ gebildeten Zuckeralkoholen mit einem Gew-%-Anteil von bis zu 3,0 Gew-% die pH-Wert-Anhebung bei gleicher Reduzierung von Zuckerstoffen im Vergleich zu einem rein enzymatischen Prozess um bis zu 0,3 pH-Einheiten geringer ausfallen kann, wobei bevorzugt beide Werte zueinander insbesondere linear korrelieren.

Damit wurde erstmals ein zuckerreduziertes Fruchtprodukt mit sensorisch ausgewogenem "Säure-Zucker-Verhältnis", also einem ausgewogenes Verhältnis saurer und süßer Geschmacksnoten, erhältlich, ohne dass es dem sonst üblichen Zusatz unerwünschter Aroma- und Farbstoffe bedurfte.

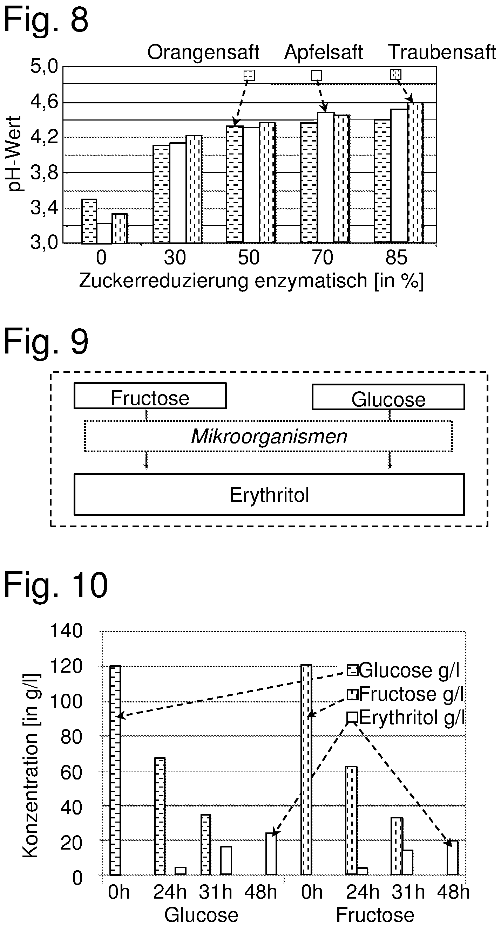

**Fig. 8** zeigt die mittlere Abhängigkeit eines ausgeglichenen Säure-Zucker-Verhältnisses verschiedener Fruchtsäfte nach einer enzymatischen Zuckerreduzierung vom pH-Wert. Demgemäß konnten in enzymatischen Prozessreaktionen sowohl geschmacklich wie auch prozesstechnisch gute Resultate erzielt werden, wenn man bei einer angestrebten Reduzierung der Zuckerstoffe um mindestens 30 Gew-% bis keiner 40 Gew-% den pH-Wert um etwa 0,8 pH-Einheiten erhöht; wenn man bei einer angestrebten Reduzierung der Zuckerstoffe um mindestens 40 Gew-% bis keiner 50 Gew-% den pH-Wert um etwa 0,9 pH-Einheiten erhöht (nicht dargestellt); wenn man bei einer angestrebten Reduzierung der Zuckerstoffe von mindestens 50 % bis kleiner 65 % den pH-Wert um etwa 1,0 pH-Einheiten erhöht; wenn man bei einer angestrebten Reduzierung der Zuckerstoffe von mindestens 65 Gew-% bis kleiner 80 Gew-% (dargestellt sind Werte bei 70 % Gew-%) den pH-Wert um etwa 1,1 pH-Einheiten erhöht; und wenn man bei einer angestrebten Zuckerreduzierung von mindestens 80 Gew-% (dargestellt sind Werte bei 85 % Gew-%) den pH-Wert um etwa 1,2 pH-Einheiten erhöht; wobei diese Werte im Einzelfall auch um 0,1 bis 0,2 pH-Einheiten höher oder niedriger ausgefallen sind.

### AB 2: Etablierung fermentativer Verfahrens zur Entfernung der Zuckerstoffe Fructose und/oder Glucose unter Bildung von Zuckeralkoholen

Nach erfolgreichem Abbau der Zuckerstoffe Saccharose und der Glucose verbleibt in den meisten Fruchtedukten als relevanter Zuckerstoff nur noch die Fructose. Obwohl erfindungsgemäß bevorzugt mit dem Enzym Glucose-Isomerase auch eine Umwandlung der Fructose in Glucose möglich ist, kann zum Abbau der Fructose auch ein nichtenzymatisches Verfahren sondern ein fermentatives Verfahren auf der Basis von Mikroorganismen eingesetzt werden, was vorteilhaft geschmacklichen Aspekten im zuckerreduzierten Fruchtprodukt durch Bildung erwünschter süßender Verbindungen Rechnung trägt.

Dabei finden im Rahmen der vorliegenden Erfindung vorzugsweise solche Mikroorganismen Verwendung, die trotz eines niedrigen pH-Werts und kaum vorhandener Stickstoffquellen (die normalerweise Grundlage für das Wachstum von Mikroorganismen ist) im Fruchtedukt und in Gegenwart fruchteigener Bakteriozide (wie z.B. Polyphenole) wachsen, insbesondere Pilze, Hefen und/oder Bakterien.

Positive Erfahrungen konnten beispielsweise mit dem Milchsäurebakterium *Leuconostoc mesenteroides* gewonnen werden. Allerdings bildet dieses laxierend wirkendes Mannit als Zuckeralkohol. Bei den gebildeten Mannitmengen von bis zu 45 g/l kam es sodenn bereits bei durchschnittlichen Verzehrmengen von 0,5 l Fruchtgetränk bei empfindlichen Personen zu einer leicht abführenden Wirkung durch die nach dem entwickelten Verfahren zuckerreduzierten Fruchtgetränke.

Daher wurden im folgenden Mikroorganismen bevorzugt, die zwar in der Lage sind, den Zuckerstoff Fructose abzubauen, bei dessen Abbau aber keine möglicherweise problematischen Produkte wie Alkohol oder Mannit entstehen, sondern vorzugsweise laxiere-freie, also nicht abführend wirkende, süßende Zuckeralkohole bilden, wie insbesondere Erythritol.

**Fig. 9** zeigt ein Reaktionsschema fermentativer Reaktionsprozesse. Dargestellt ist, wie der Abbau der Zuckerstoffe Fructose und/oder Glucose mittels Mikroorganismen bei gleichzeitiger Bildung von Erythritol erfolgen kann.

Diesbezüglich positive Erfahrungen konnten beispielsweise mit dem Pilz *Monitiella tomentosa* oder mit der Hefe *Candida magnoliae* gewonnen werden.

**Fig. 10** zeigt die fermentative Reduzierung der Zuckerstoffe Glucose (links) und Fructose (rechts) mittels des Pilzes *Moniliella tomentosa* unter Bildung von Erythritol. Erkennbar ist, wie nach 24 h die Bildung des Zuckeralkohols Erythritol gerade erst beginnt, aber bereits nach 48 Stunden die Zuckerstoffe vollständig abgebaut sind.

Dabei bewirkt der Einsatz des Pilzes *Moniliella tomentosa* vorteilhaft, dass von einer beliebigen Menge an Zuckerstoffen (= 100%) bereits etwa die Hälfte (= 50 %) von den Mikroorganismen "verbraucht" werden und die anderen 50 % zu süßenden, kalorienreduzierenden Verbindungen im Verhältnis von etwa 2 : 1 umgewandelt werden, was bezogen auf die eingesetzte Menge an Zuckerstoffen (= 100 %) eine effektive Zuckerstoff- bzw. Kalorienreduzierung von ursprünglich 100 % um 75 % auf 25 % im angestrebten Fruchtprodukt zum Vorteil hat.

Ebenso wie bei rein enzymatischen Reaktionsprozessen (siehe oben) wurde auch beim fermentativen Reaktionsprozess der pH-Wert im zuckerreduzierten Fruchtprodukt auf einen vorgegebenen höheren Wert gegenüber dem pH-Wert im Fruchtedukt eingeregelt, um ein ausgeglichenes Säure-Zucker-Verhältnis zu erreichen, was die Voraussetzung einer geschmacklichen Akzeptanz beim Verbraucher darstellt.

Durch die Bildung süßschmeckender Verbindungen wie Erythritol konnte aber bei gleicher Reduzierung von Zuckerstoffen im Vergleich zu rein enzymatischen Reaktionsprozess bei fermentativ gebildeten Zuckeralkoholen mit einem Gew-%-Anteil von bis zu 3,0 Gew-% die erfindungsgemäße pH-Anhebung um bis zu 0,3 pH-Einheiten geringer ausfallen, wobei bevorzugt beide Werte zueinander insbesondere linear korrelieren.

Die Abhängigkeit eines ausgeglichenen Säure-Zucker-Verhältnisses vom pH-Wert bei der fermentativen Zuckerreduzierung und Erythritol-Produktion beispielsweise in Apfelsaft verdeutlicht sich auch anhand der nachstehenden Tabelle:

| Zuckerreduzierung in % | Erythritol-Gehalt in % | pH ausgeglichenes Zucker-Säure Verhältnis |
|---|---|---|
| 30 | 0,7 | 3,9 |
| 50 | 1,4 | 4,2 |
| 70 | 1,8 | 4,3 |
| 85 | 2,2 | 4,5 |

### AB 3: Kombination enzymatischer und fermentativen Verfahren

Das erfindungsgemäße Verfahren zur biotechnologischen Reduzierung von Zuckerstoffen in Fruchtedukten zwecks Erhalts zuckerreduzierter Fruchtprodukte, wird sich in der Praxis auch durch die Kombination enzymatischer und fermentativer Reaktionsprozesse auszeichnen. Derartige Kombinationsverfahren machen eine Art Baukasten- oder Plattformtechnologie verfügbar, mit deren Hilfe in vielen Lebensmitteln alle dort relevanten Fraktionen an Zuckerstoffen mittels biotechnologischer Verfahren entfernt werden können.

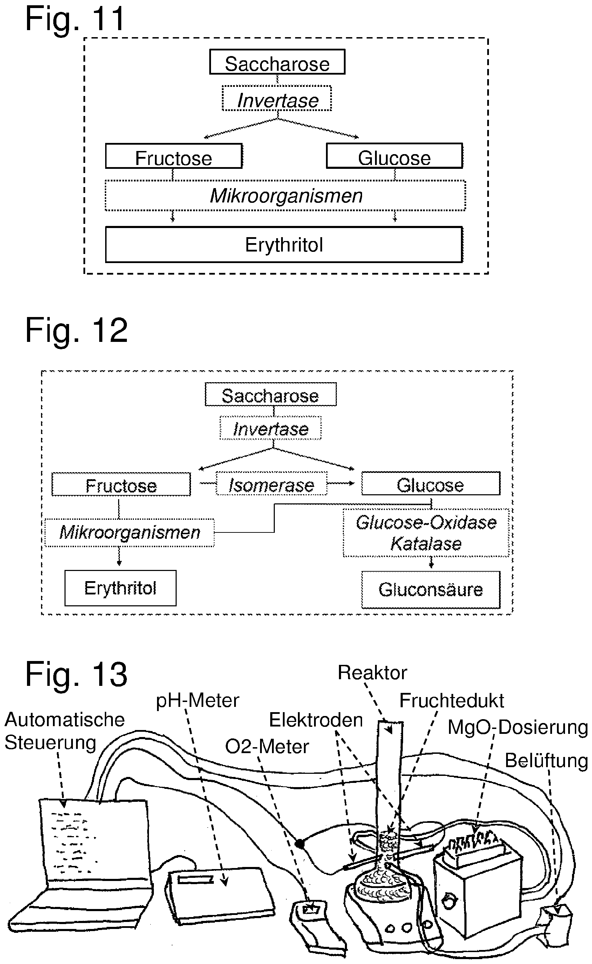

**Fig. 11** zeigt beispielhaft ein Reaktionsschema enzymatisch und fermentativ kombinierter Reaktionsprozesse mit einem fermentativen Schwerpunkt. Dargestellt ist der Abbau der Zuckerstoffe Fructose und/oder Glucose mittels Mikroorganismen bei gleichzeitiger Bildung von Erythritol und wahlweisem Abbau des Zuckerstoffes Saccharose durch Einsatz des Enzyms Invertase.

**Fig. 12** zeigt beispielhaft ein Reaktionsschema der Kombination aller zuvor beschriebenen enzymatischen und fermentativen Reaktionsprozesse mit beliebig wählbaren (enzymatischen und/oder fermentativen) Schwerpunkten. Mit diesen Verfahren können alle in Fruchtedukten enthaltene Zuckerstoffe (wie insbesondere Saccharose, Fructose und/oder Glucose) in kombinierten Reaktionsprozessen enzymatisch und/oder fermentativ abgebaut werden und dennoch zugleich süßende Stoffe im zuckerreduzierten Fruchtprodukt vorteilhaft erhalten bleiben. Zusätzlich können in einem Fermentationsschritt kalorienreduzierte süßschmeckende Verbindungen wie insbesondere Erythritol gebildet werden.

### AB 4: Apparativer Aufbau und Prozesskontrolle

**Fig. 13** zeigt beispielhaft an Hand eines apparativen Labor-Aufbaus, wie sowohl in den enzymatischen als auch in etwaigen fermentativen Reaktionsprozessen eine ausgeklügelte, aber kostengünstige Mess- und Regeltechnik von Vorteil ist, um einen reproduzierbaren und sicheren Prozessablauf zu gewährleisten, welcher vorzugsweise in einem Batch-Verfahren erfolgt. Dargestellt ist, wie die für einen Einzelprozess also auch einen Kombinationsprozess benötigte Reaktortechnik nebst eingesetzten Sensoren (pH und O2-Elektroden) und, bevorzugt automatischen, Steuerungs- und Regelungstechnik ohne Rückgriff auf teure oder aufwendige Gerätschaften auskommt.

So kann beispielsweise eine Belüftung über eine einfache Fritte für den Eintrag des für den Abbau der Glucose zu Gluconsäure benötigten Sauerstoffs in eine Vorrichtung (Reaktor) zur Durchführung des Verfahrens ausreichend sein. Alternativ oder kumulativ dazu können auch Mittel zum Einblasen von Sauerstoff in die Vorrichtung zur Durchführung des Verfahrens vorgesehen sein.

Zur pH-Regelung werden als Säureregulator bevorzugt Magnesiumoxid (MgO) und/oder Magnesiumoxidsuspensionen eingesetzt, da diese in vorteilhafter Weise keinen negativen Einfluss auf den Geschmack der angestrebten zuckerreduzierten Fruchtprodukte haben.

Die dargestellte Labor-Vorrichtung zur Durchführung eines Verfahrens nach der Erfindung umfasst beispielsweise einen 3 Liter Reaktor mit einer pH-Elektrode, einer Sauerstoffelektrode und einer Fritte zur Belüftung mit reinem Sauerstoff. Natürlich kann auch der 20 % Anteil des Sauerstoffs in Luft genutzt werden, nur sinkt dann entsprechend die Prozessgeschwindigkeit. Vorzugsweise werden während der Reaktionsprozesse die Fruchtedukte im Reaktor mittels eines Magnet- oder vergleichen Rührers gerührt, um eine gleichmäßige Sauerstoff- und MgO-Verteilung zu gewährleisten. Magnesiumoxid (MgO) ist schwerlöslich und wird deshalb bevorzugt als 10%-ige Suspension, beispielsweise mit Hilfe einer Schlauchpumpe, zu dosiert. Über eine automatische Steuerung, beispielsweise ein computergesteuertes Regelsystem, lassen sich während der Reaktionsprozesse der pH-Wert und die Sauerstoffsättigung automatisch einregeln:
Nachdem im enzymatischen Reaktionsprozess mittels Glucose-Oxidase Sauerstoff verbraucht wird und durch die gebildete Gluconsäure der pH-Wert sinkt, wird beispielsweise über ein Ventil die Sauerstoffzufuhr sowie beispielsweise über eine Pumpe die Zudosierung des Säureregulators Magnesiumoxid gesteuert. Die Sauerstoff-Kontrolle erfolgt vorzugsweise mittels eines O₂-Meters, die pH Kontrolle vorzugsweise mittels eines pH-Meters.

Letztere ist auch in einem dem enzymatischen Reaktionsprozess folgenden Fermentationsschritt vorteilhaft, da auch insbesondere bei Milchsäurebakterien mit dem Fructose-Abbau eine pH-Wert Absenkung einhergeht, welcher durch Zugabe z.B. von Natriumhydroxid (NaOH) entgegen gewirkt werden kann. Soweit der fermentative Schritt anaerob abläuft, ist hier eine Sauerstoffzugabe nicht (mehr) notwendig.

Unabhängig davon, inwieweit aus insbesondere prozessbeschleunigenden Aspekten eine pH-Wert-Kontrolle auch während der enzymatischen und/oder fermentativen Reaktionsprozesse im Fruchtedukt Anwendung finden, erfolgt der erfindungsgemäße Regelprozess für den pH-Wert im zuckerreduzierten Fruchtprodukt bevorzugt mit Hilfe eines Mittels zum Einregeln eines vorgegebenen pH-Wertes im Fruchtprodukt durch Zudosierung eines Säureregulators auf einem gegenüber dem pH-Wert im Fruchtedukt erhöhten Wert. Vorzugsweise, mittels des pH-Meters beispielsweise, wird dazu zunächst der pH-Wert im Fruchtedukt bestimmt, und anschließend die Zudosierung eines Säureregulators solange überwacht, wie enzymatische und/oder fermentative Reaktionsprozesse ablaufen, so dass auch nach Beendigung enzymatischer und/oder fermentativer Reaktionsprozesse sichergestellt ist,
- dass bei einer Reduzierung der Zuckerstoffe um mindestens 30 Gew-% bis kleiner 40 Gew-% der pH-Wert zwischen 0,6 und 1,0 pH-Einheiten erhöht ist; oder
- dass bei einer Reduzierung der Zuckerstoffe um mindestens 40 Gew-% bis kleiner 50 Gew-% der pH-Wert zwischen 0,7 und 1,1 pH-Einheiten erhöht ist; oder
- dass bei einer Reduzierung der Zuckerstoffe um mindestens 50 Gew-% bis kleiner 65 Gew-% der pH-Wert zwischen 0,8 und 1,2 pH-Einheiten erhöht ist; oder
- dass bei einer Reduzierung der Zuckerstoffe um mindestens 65 Gew-% bis kleiner 80 Gew-% der pH Wert zwischen 0,9 und 1,3 pH Einheiten erhöht ist; oder
- dass bei einer Reduzierung der Zuckerstoffe um mindestens 80 Gew-% der pH-Wert zwischen 1,0 und 1,4 pH-Einheiten erhöht ist;
wobei die vorgenannten pH-Werte jeweils auch um bis zu 0,1 oder bis zu 0,2 pH-Einheiten höher oder niedriger ausfallen können; und/oder
wobei bei fermentativ gebildeten Zuckeralkoholen mit einem Gew-%-Anteil von bis zu 3,0 Gew-% die pH-Wert-Anhebung bei gleicher Reduzierung von Zuckerstoffen im Vergleich zu einem rein enzymatischen Prozess um bis zu 0,3 pH-Einheiten geringer ausfallen kann, wobei bevorzugt beide Werte zueinander insbesondere linear korrelieren.

### AP5: Fruchtproduktbeispiel

Die Untersuchung des Gesamtprozesses wurde im Folgenden am Beispiel eines Ananas Direktsaftes durchgeführt. Dieser wurde im ersten Schritt in einem Reaktor mit den Enzymen Glucose Oxidase, Katalase und Invertase versetzt und unter Einstellung und Regelung eines Sauerstoffgehalts von 30 % mittels Magnetventil und Computersteuerung wurde der enzymatische Reaktionsprozess gestartet.

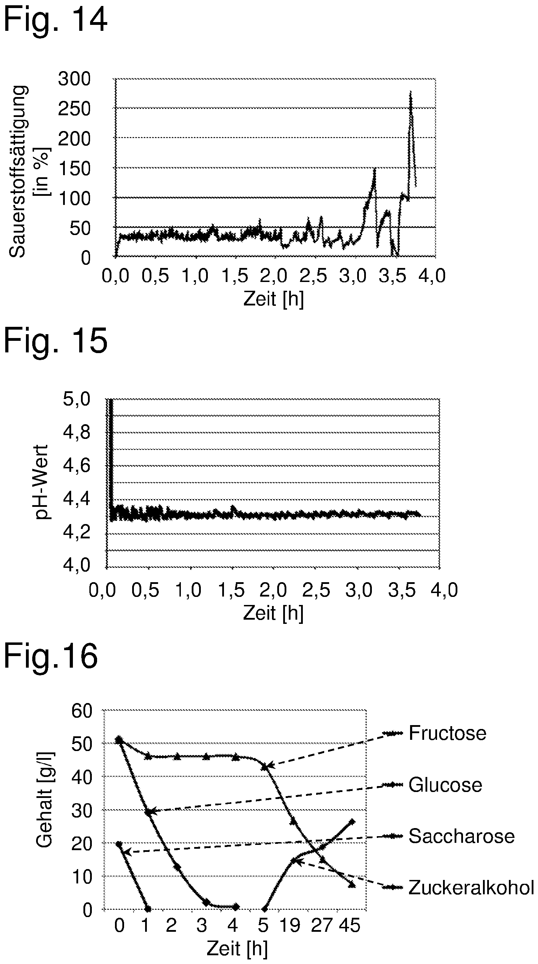

**Fig. 14** zeigt die Sauerstoffsättigung im Verlauf des enzymatischen Abbaus der Zuckerstoffe Saccharose und Glucose in dem Ananas Direktsaft. Erkennbar ist, dass die Regelung in den ersten 3 Stunden sehr gut funktionierte. Der Sauerstoffgehalt nahm erst wieder zu, als die gesamte Glucose im Ananassaft vollständig abgebaut war.

Auch der pH-Wert wurde während des enzymatischen Prozessschrittes in gewünschter Weise auf einen höheren pH-Wert als im Fruchtedukt vorgelegen eingeregelt. Nach dessen Einstellung auf beispielsweise pH 4,3 zu Beginn des Prozesses gelang es mit dem Regelprozess, diesen innerhalb eines Fensters von nur 0,5 pH Einheiten zu halten:
**Fig. 15** zeigt den pH Verlauf während des enzymatischen Abbaus der Zuckerstoffe Saccharose und Glucose im Ananas Direktsaft.

**Fig. 16** schließlich zeigt die prinzipielle Veränderung der Konzentration der Zuckerstoffe (Saccharose und Glucose) während des enzymatischen Abbaus und der Bildung von Zuckeralkohol während des fermentativen Abbaus von Fructose in einem Ananas Direktsaft.

Diese beschriebene Regelung ermöglichte zuverlässig den schnellen enzymatischen Abbau der Zuckerstoffe Saccharose und Glucose innerhalb von nur 4 Stunden in dem eingesetzten Ananassaft.

Durch den ab der fünften Stunde erfolgten Einsatz geeigneter Mikroorganismen gelang binnen weiterer 40 Stunden auch ein fast vollständiger Abbau des Zuckerstoffes Fructose unter Bildung eines Zuckeraustauschstoffes.

Somit ist auch ein Kombinationsverfahren nach der Erfindung realisierbar und in der Lage, unter schonenden Temperaturbedingungen (zwischen z.B. nur 20 bis 30 °C) innerhalb von 45 Stunden mehr als 95 % alle wesentlichen in einem Direktsaft enthaltenen Zuckerstoffe (Saccharose, Glucose und Fructose) abzubauen.

Die Herstellung zuckerreduzierter Fruchtprodukte durch das hier vorgestellte biotechnologische Verfahren bietet somit eine Möglichkeit, sich mit innovativen Fruchtprodukten über ein Alleinstellungsmerkmal auf dem Markt zu etablieren. Momentan gibt es nämlich kein zuckerreduziertes Fruchtprodukt, das auf der einen Seite die in zerkleinerten Früchten als Fruchtedukte enthaltenen gesunden natürlichen Inhaltsstoffe beinhaltet, gleichzeitig stark zuckerreduziert ist und zudem ein geschmacks-sensorisch ausgewogenes Säure-Zucker-Verhältnis aufweist.

Mittels der vorliegenden Erfindung aber sind insbesondere zuckerreduzierte Fruchtprodukte erhältlich wie Fruchtpürees oder Fruchtzubereitungen oder Fruchtpulver oder Ganzfruchtgetränke (Smoothies) oder Obst- und/oder Gemüsesäfte (gleich ob unverdünnt als Direktsaft oder rückverdünnt als Fruchtsaft aus Fruchtsaftkonzentrat abgefüllt) oder vergleichbare als alkoholfrei kennzeichenbare Fruchtgetränke; wobei der pH-Wert im zuckerreduzierten Fruchtprodukt erfindungsgemäß auf einen vorgegebenen höheren Wert gegenüber dem pH-Wert im Fruchtedukt eingeregelt ist.

## Patentansprüche

1. Verfahren zur biotechnologischen Reduzierung von Zuckerstoffen in Fruchtedukten zwecks Erhalts zuckerreduzierter Fruchtprodukte, durch
- enzymatische Reaktionsprozesse
**gekennzeichnet durch**
- einen Regelprozess, mit dem der pH-Wert im zuckerreduzierten Fruchtprodukt so auf einen vorgegebenen höheren Wert gegenüber dem pH- Wert im Fruchtedukt eingeregelt wird,
- dass bei einer Reduzierung der Zuckerstoffe um mindestens 30 Gew-% bis kleiner 40 Gew-% der pH-Wert zwischen 0,6 und 1,0 pH-Einheiten erhöht ist; oder
- dass bei einer Reduzierung der Zuckerstoffe um mindestens 40 Gew-% bis kleiner 50 Gew-% der pH-Wert zwischen 0,7 und 1, 1 pH-Einheiten erhöht ist; oder
- dass bei einer Reduzierung der Zuckerstoffe um mindestens 50 Gew-% bis kleiner 65 Gew-% der pH-Wert zwischen 0,8 und 1,2 pH-Einheiten erhöht ist; oder
- dass bei einer Reduzierung der Zuckerstoffe um mindestens 65 Gew-% bis kleiner 80 Gew-% der pH Wert zwischen 0,9 und 1,3 pH Einheiten erhöht ist; oder
- dass bei einer Reduzierung der Zuckerstoffe um mindestens 80 Gew-% der pH-Wert zwischen 1,0 und 1,4 pH-Einheiten erhöht ist;
wobei die zuckerreduzierten Fruchtprodukte Ganzfruchtgetränke oder Obst- und/oder Gemüsesafte sind,
(a) **gekennzeichnet durch** eine
- enzymatische Umwandlung von Saccharose in Glucose und Fructose mittels Invertase; und/oder
- enzymatische Umwandlung von Fructose zu Glucose mittels Glucose- Isomerase und
(b) **gekennzeichnet durch** einen
- enzymatischen Abbau von Glucose unter Bildung von Gluconsäure und Wasserstoffperoxid mittels Glucose-Oxidase
- bei gleichzeitigem Einsatz von Katalase, um entstandenes Wasserstoffperoxid in Wasser und Sauerstoff umzuwandeln.

2. Verfahren nach Anspruch 1, **dadurch gekennzeichnet, dass** während der enzymatischen Reaktionsprozesse mittels Glucose-Oxidase und Katalase benötigter Sauerstoff zugeführt wird, wobei die Sauerstoffzufuhr vorzugsweise auf Sättigungen zwischen 5 % und 60 %, insbesondere zwischen 10 % und 40 %, besonders bevorzugt zwischen 30 % und unter 40 %, eingeregelt wird.

3. Verfahren nach Anspruch 2, **dadurch gekennzeichnet, dass** der Sauerstoff gleichmäßig in eine Vorrichtung zur Durchführung des Verfahrens eingeblasen wird.

4. Verfahren nach einem der vorherigen Ansprüche, **dadurch gekennzeichnet, dass** das Einregeln auf einen höheren pH-Wert durch Zugabe geschmacksneutraler Salze und/oder Salzsuspensionen, wie insbesondere Magnesiumoxid und/oder Magnesiumoxidsuspension, erfolgt.

## Claims

1. Method for biotechnological reduction of sugars in progeny starting materials for the purpose of obtaining sugar-reduced progeny products, by
- enzymatic reaction processes
**characterized by**
- a regulation process by means of which the pH in the sugar-reduced progeny product is adjusted to a specified higher value compared to the pH in the progeny starting material such
- that, in the case of a reduction of the sugars by at least 30% by weight to less than 40% by weight, the pH has been increased between 0.6 and 1.0 pH units; or
- that, in the case of a reduction of the sugars by at least 40% by weight to less than 50% by weight, the pH has been increased between 0.7 and 1.1 pH units; or
- that, in the case of a reduction of the sugars by at least 50% by weight to less than 65% by weight, the pH has been increased between 0.8 and 1.2 pH units; or
- that, in the case of a reduction of the sugars by at least 65% by weight to less than 80% by weight, the pH has been increased between 0.9 and 1.3 pH units; or
- that, in the case of a reduction of the sugars by at least 80% by weight, the pH has been increased between 1.0 and 1.4 pH units;
wherein the sugar-reduced progeny products are wholeprogeny beverages or fruit and/or vegetable juices,
(a) **characterized by** an
- enzymatic conversion of sucrose into glucose and fructose by means of invertase; and/or
- enzymatic conversion of fructose to glucose by means of glucose isomerase and
(b) **characterized by** an
- enzymatic degradation of glucose to form gluconic acid and hydrogen peroxide by means of glucose oxidase
- with simultaneous use of catalase in order to convert resultant hydrogen peroxide into water and oxygen.

2. Method according to Claim 1, **characterized in that** necessary oxygen is supplied during the enzymatic reaction processes by means of glucose oxidase and catalase, wherein the supply of oxygen is preferably adjusted to saturations between 5% and 60%, especially between 10% and 40% and particularly preferably between 30% and below 40%.

3. Method according to Claim 2, **characterized in that** the oxygen is evenly blown into an apparatus for carrying out the method.

4. Method according to any of the preceding claims, **characterized in that** the adjustment to a higher pH is effected by addition of tasteless salts and/or salt suspensions, such as, in particular, magnesium oxide and/or magnesium oxide suspension.

## Revendications

1. Procédé de réduction biotechnologique des sucres dans des matières premières de fruits dans le but d'obtenir des produits de fruits à teneur réduite en sucres, par
- des procédés enzymatiques
**caractérisé par**
- un processus de régulation, à l'aide duquel le pH du produit de fruits à teneur réduite en sucres est ajusté à une valeur prédéfinie supérieure au pH de la matière première de fruits,
- en ce que, pour une réduction des sucres d'au moins 30 % en poids à moins de 40 % en poids, le pH est élevé de 0,6 à 1,0 unité de pH ; ou
- en ce que, pour une réduction des sucres d'au moins 40 % en poids à moins de 50 % en poids, le pH est élevé de 0,7 à 1,1 unité de pH ; ou
- en ce que, pour une réduction des sucres d'au moins 50 % en poids à moins de 65 % en poids, le pH est élevé de 0,8 à 1,2 unité de pH ; ou
- en ce que, pour une réduction des sucres d'au moins 65 % en poids à moins de 80 % en poids, le pH est élevé de 0,9 à 1,3 unité de pH ; ou
- en ce que, pour une réduction des sucres d'au moins 80 % en poids, le pH est élevé de 1,0 à 1,4 unité de pH ;
les produits de fruits à teneur réduite en sucres étant des boissons à base de fruits entiers ou des jus de fruits et/ou de légumes,
(a) **caractérisé par**
- une conversion enzymatique du saccharose en glucose et en fructose à l'aide d'une invertase ; et/ou
- une conversion enzymatique du fructose en glucose à l'aide d'une glucose-isomérase, et
(b) **caractérisé par**
- une dégradation enzymatique du glucose avec formation d'acide gluconique et de peroxyde d'hydrogène à l'aide d'une glucose-oxydase
- avec utilisation simultanée d'une catalase, pour convertir le peroxyde d'hydrogène formé en eau et oxygène.

2. Procédé selon la revendication 1, **caractérisé en ce que**, au cours des processus enzymatiques à l'aide d'une glucose-oxydase et d'une catalase, on amène l'oxygène nécessaire, l'apport d'oxygène étant de préférence ajusté à des saturations entre 5 % et 60 %, en particulier entre 10 % et 40 %, d'une manière particulièrement préférée entre 30 % et moins de 40 %.

3. Procédé selon la revendication 2, **caractérisé en ce que** l'oxygène est uniformément insufflé dans un dispositif destiné à la mise en œuvre du procédé.

4. Procédé selon l'une des revendications précédentes, **caractérisé en ce que** l'ajustement à un pH plus élevé est réalisé par addition de sels à goût neutre et/ou de suspensions de sels tels en particulier l'oxyde de magnésium et/ou une suspension d'oxyde de magnésium.
